# EUROPÄISCHE PATENTANMELDUNG

(11) **EP 2 442 202 A2**
(43) Veröffentlichungstag der Anmeldung: **18.04.2012**
(21) Anmeldenummer: 11160614.1
(22) Anmeldetag: 31.03.2011
(51) Int. Cl.: G05D 11/13, B01F 1/00, B65B 3/00, F17C 5/06

(54) **Vorrichtung zum Befüllen eines Spritzenkörpers mit einem Gasgemisch zum anschließenden Injizieren eines Gemisches aus dem Gasgemisch und einem Medikament in den menschlichen oder tierischen Körper**

(30) Priorität: 09.04.2010 DE 202010005131 U
(71) Anmelder: HOISS, Jakob, D-80637 München (DE)
(72) Erfinder: HOISS, Jakob, D-80637 München (DE)
(74) Vertreter: Dreiss

(57) **Zusammenfassung**

Eine Vorrichtung (10) zum Befüllen eines Spritzenkörpers (43) mit einem Gasgemisch zum anschließenden Injizieren eines Gemisches aus dem Gasgemisch und einem Medikament in den menschlichen oder tierischen Körper in Anwendung für Diagnose und/oder Therapie ist mit zwei in getrennten Leitungssträngen (12, 13) angeordneten Ventilanordnungen aus jeweils einem Feinregulierventil (14, 15) und in Reihe geschaltetem Schließventil (16, 17) in einem transportablen Behältnis (11) versehen. Dabei ist das jeweils eine Ende der Leitungsstränge (12, 13) mit jeweils einem von zwei Druckgasquellen und das jeweils andere Ende der Leitungsstränge (12, 13) mit einem mit dem betreffenden Gas zu füllenden Spritzenkörper (43) an der Außenseite des Behältnisses (11) lösbar verbindbar.

## Beschreibung

Die vorliegende Erfindung bezieht sich auf eine Vorrichtung zum Befüllen eines vorzugsweise Spritzenkörpers mit einem Gasgemisch zum anschließenden Injizieren eines Gemisches aus dem Gasgemisch und einem Medikament in den menschlichen oder tierischen Körper, nach dem Oberbegriff des Anspruchs 1.

Für die Anwendung in der Diagnose und/oder Therapie von Teilen des menschlichen oder tierischen Körpers ist es in der Medizin bekannt, bestimmte Gase einschließlich steriler Luft zu verwenden. Beispielsweise erfolgt dies bei einer festen Gaszusammensetzung entsprechend der GB 422 307 A oder der EP 0 564 505 B1.

Aufgabe der vorliegenden Erfindung ist es, eine Vorrichtung der eingangs genannten Art zu schaffen, mit der es möglich ist, in einfacher Weise ein Gasgemisch zum anschließenden Mischen - möglich ist ohne Eintrag von Fremdgasen oder Fremdstoffen - mit einem Medikament zu schaffen, bei dem die verwendeten vorzugsweise zwei Gase in einem wählbaren Volumenverhältnis gemischt werden können.

Zur Lösung dieser Aufgabe sind bei einer Vorrichtung der genannten Art die im Anspruch 1 angegebenen Merkmale vorgesehen.

Durch die erfindungsgemäßen Maßnahmen ist erreicht, dass entsprechend den von Hand eingestellten Feinregulierventilen bzw. deren Durchflussmengen pro Zeiteinheit in einfacher Weise ein beobachtbares Füllen des Spritzenkörpers mit einem entsprechenden Mischungsvolumenverhältnis der beiden Gase erreichbar ist. Eine derartige Vorrichtung ist sowohl in ihrem Aufbau als auch in ihrer Handhabung äußerst einfach wirkend und robust sowie einfach zu bedienen.

Mit den Merkmalen nach Anspruch 2 ist eine einfache Voreinstellung der Feinregulierventile möglich.

Weitere vorteilhafte Ausgestaltungen ergeben sich aus den Merkmalen nach einem oder mehreren der Ansprüche 3 bis 5, wonach zunächst das jeweils einem Durchfluss eines bestimmten Gases zugeordnete Schließventil ausgewählt und geöffnet und nach entsprechender Zeit wieder geschlossen wird.

Für eine einfache Handhabung und ein wahlweises Einsetzen der Vorrichtung sind die Merkmale nach Anspruch 6 vorgesehen. Ein derartiger in einfacher Weise zu handhabender Aktenkoffer kann gemäß den Merkmalen nach Anspruch 7 und/oder 8 und/oder 9 ausgestaltet sein.

In vorteilhafter Weise sind die Merkmale nach Anspruch 10 vorgesehen.

Für bestimmte Anwendungen besteht gemäß den Merkmalen des Anspruchs 11 das Gasgemisch aus zwei bestimmten in einfacher Weise zur Verfügung zu stellenden Gasen.

Weitere Einzelheiten der Erfindung sind der folgenden Beschreibung zu entnehmen, in der die Erfindung anhand des in der Zeichnung dargestellten Ausführungsbeispieles näher beschrieben und erläutert ist. Es zeigen:
- Figur 1: in schematischer Blockdarstellung eine Vorrichtung zur Herstellung eines Gasgemisches gemäß einem bevorzugten Ausführungsbeispiel vorliegender Erfindung,
- Figur 2: in vergrößerter Darstellung einen teilweise abgebrochenen Ausschnitt gemäß Rechteck II der Figur 1 und
- Figur 3: eine der Figur 1 entsprechende Darstellung, jedoch gemäß einem modifizierten Ausführungsbeispiel vorliegender Erfindung.

In der Zeichnung ist eine Vorrichtung 10 bzw. 10' zum gezielten Weiterleiten von vorzugsweise zwei unterschiedlichen Gasen, insbesondere Sauerstoff (O₂) und Kohlendioxid (CO₂) in zeitlicher Reihenfolge in einen Aufnahmekörper 43, insbesondere einen Spritzenkörper, und dabei zum Mischen der beiden Gase in dem Aufnahmekörper dargestellt. Dieses vom Aufnahmekörper 43 aufgenommene Gasgemisch aus einem gewählten und vorbestimmten Volumenverhältnis der beiden Gase Sauerstoff und Kohlendioxid wird danach für die weitere Verwendung mit einem Medikament gemischt, welche Gas-/Medikamentenmischung in den menschlichen oder tierischen Körper in Anwendung für Diagnose und/oder Therapie injiziert werden kann.

Die Vorrichtung 10, deren Bauteile in einem transportablen Behältnis 11, wie beispielsweise einem mit einem Deckel versehenen Aktenkoffer, vorzugsweise aus einem Leichtmetall-Verbund, untergebracht ist, besitzt zwei getrennt voneinander vorgesehene, wirkungsmäßig parallele Leitungsstränge 12, 13, von denen jeder Leitungsstrang 12, 13 mit einem Feinregulierventil 14 bzw. 15 sowie einem damit in Reihe geschalteten Schließventil 16 bzw. 17 versehen ist. Das Feinregulierventil 14, 15, das in Strömungsrichtung A eingangsseitig der Vorrichtung 10 und vor dem Schließventil 16 bzw. 17 angeordnet ist, ist hinsichtlich seines Strömungsquerschnitts einstellbar und festlegbar. Das Schließventil 16, 17, das in Strömungsrichtung A ausgangsseitig der Vorrichtung 10 angeordnet ist, ist mit einem beispielsweise elektromagnetisch angetriebenen Verschluss 18 bzw. 19 versehen. Mit einem nicht dargestellten Wählschalter sind die Verschlussantriebe 18 und 19 derart verbunden, dass jeweils für ein abwechselndes Öffnen und Schließen einer der beiden aktiviert und der andere deaktiviert ist. Mit anderen Worten, nur jeweils derjenige Leitungsstrang 12, 13, in dem der Verschlussantrieb 18, 19 des Schließventils 16, 17 aktiviert ist, kann mittels eines weiteren Schalters 21, bspw. in Form eines Rasttasters, zum Durchfluss freigegeben werden. Der Schalter 21 wird jeweils zum Öffnen und zum Sperren des Durchflusses gedrückt, wobei die Durchflusszeit von Hand dadurch bestimmt wird, dass das Füllen des Spritzenkörpers 43 anhand von dessen Skalierung beobachtet wird. Hierzu wird der Öffnungsquerschnitt der Feinregulierungsventile 14 und 15 so eingestellt, dass eine akzeptable, mit dem Auge beobachtbare Füllgeschwindigkeit von bspw. 10 ml/10 sec entsteht, wobei die Öffnungsquerschnitte aufgrund der unterschiedlichen Molekülgröße der verwendeten Gase entsprechend unterschiedlich sind.

Beim Ausführungsbeispiel wird der skalierte Spritzenkörper mit einer Gasmischung aus 70% CO₂ und 30% O₂ ganz oder teilweise befüllt.

Bei der Vorrichtung 10 sind die eingangsseitigen Enden der Leitungsstränge 12 und 13 durch eine Seitenwand 23 des transportablen Behältnisses 11 von innen nach außen durchgeführt und außenseitig als Steckkupplung 25, 26 ausgebildet.

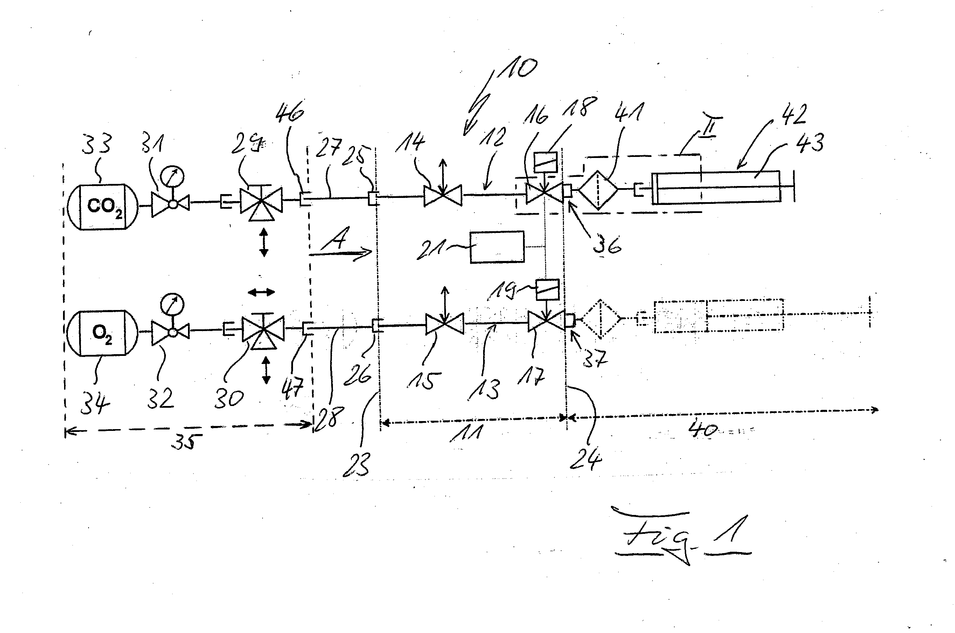

An die Steckkupplung 25 bzw. 26 kann gemäß Figur 1 ein Schlauch 27 bzw. 28 lösbar steckbar verbunden werden, der über ein Entlastungsventil 29 bzw. 30 und einem mit einem Manometer versehenen Reduzierventil 31 bzw. 32 mit einer Druckgasquelle, vorzugsweise einer Druckgasflasche 33 bzw. 34 für Kohlendioxid (CO₂) einerseits bzw. Sauerstoff (O₂) andererseits verbunden ist. Die Entlastungsventile 29, 30 können auch entfallen, falls die Entlastung der jeweiligen druckbehafteten Leitung über die Vorrichtung 10 selbst durchgeführt wird. Diese Einheit 35 aus den Flaschen 33 und 34 und den Bauteilen 31 und 32 und ggf. 29 und 30 kann ebenfalls in einem transportablen Behältnis, wie einem Koffer, untergebracht sein, dessen einendige Steckkupplung 46, 47 über die Leitung 27, 28 mit der Steckkupplung 25, 26 der Vorrichtung 10 verbindbar ist.

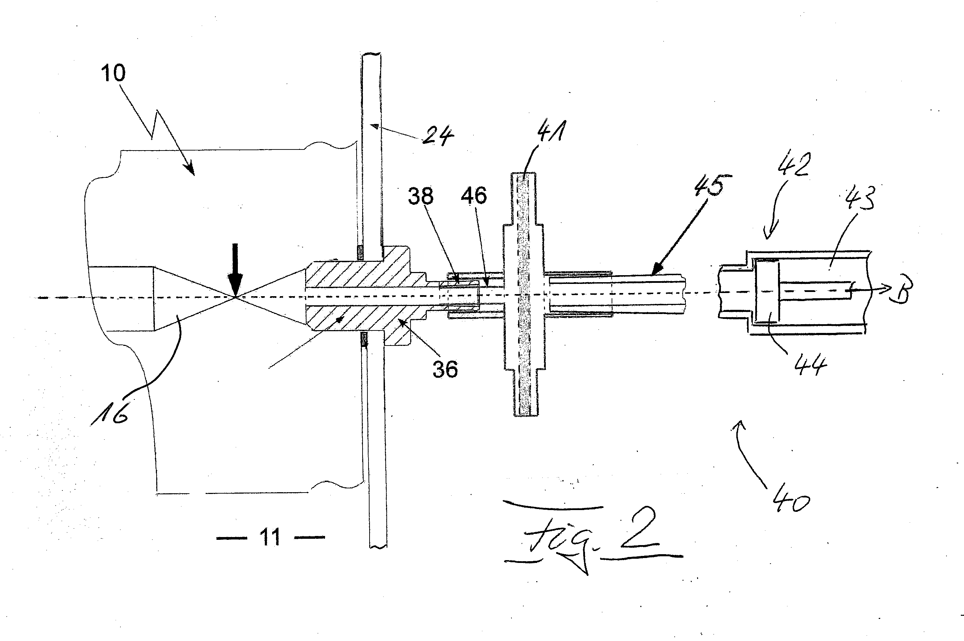

Die anderen Enden der Leitungsstränge 12 und 13 sind durch die andere Seitenwand 24 des transportablen Behältnisses 11 durchgeführt. Beim Ausführungsbeispiel sind gemäß Figur 2 die Ausgänge der Schließventile 16, 17 unmittelbar mit einem Anschlussadapter 36 bzw. 37 verbunden, der in die Ausgangsöffnung des Schließventils 16 bzw. 17 einenends eingebracht und andernends mit einem innen konisch verlaufenden Endstück 38 versehen, außenseitig herausragt, und in der Seitenwand 24 insbesondere gas- und feuchtigkeitsdicht gehalten ist. Die direkte Anordnung der Ausgänge der Schließventile 16, 17 bewirkt, dass nur ein kleines innere Volumen zwischen dem Ventil und dem später aufgebrachten Sterilfilter 41 verbleibt. Damit wird erreicht, dass beim Spülen der Adapter 36 bzw. 37 nur wenig Spülgas benötigt wird. Das Spülen ist nötig, um das innere Volumen zwischen Schließventil 17 bzw. 18 und dem Inneren des Sterilfiltergehäuses 41 vor Aufsetzen der Spritze 42 von Fremdgasen und Fremdstoffen frei zu blasen und das Sterilfilter 41 auch auf der der Spritze zugewandten Seite durch den Gasüberdruck des medizinischen Reinstgases hier z.B. O₂ oder CO₂solange innen sauber und steril zu belassen, bis die Spritze 42 an das Sterilfilter 41 angekoppelt wird. Der Anschlussadapter 36, 37 ist im Wesentlichen durch ein geradliniges Röhrchen gebildet, welches innen ein kurzes Stück 38 als Konus (auch Dichtkegel genannt) ausgebildet ist. Dies dient dazu, dass das Sterilfilter 41 schnell und einfach auswechselbar ist und durch den Konus 38 durch einfaches Aufschieben gasdicht verbunden werden kann. Hierzu besitzten die Anschlussadapter 36 bzw. 37 das Endstück 38, das die Gasdichtigkeit nach Verbindung (einfaches Einschieben) mit dem Anschlussrohr 46 des Sterilfilters 41 herstellt.

Das außerhalb des tragbaren Behältnisses 11 angeordnete freie Ende 38 des Anschlussadapters 36, 37 ist derart ausgebildet, dass eine Einheit 40 aus Sterilfilter 41 und Spritze 42 mit dem Aufnahmekörper 43 wahlweise auf den Anschlussadapter 36 oder den Anschlussadapter 37 gasdicht und abnehmbar aufgesteckt werden kann.

Beim Ausführungsbeispiel wird die Einheit 40 zunächst auf den Anschlussadapter 36 aufgebracht, so dass nach Betätigen des Wahlschalters und Drücken des Rasttasters 21 zum Öffnen des Schließventils 16 der Aufnahmekörper 43 der Spritze 42 mittels der Vorrichtung 10 mit sterilem Kohlendioxid beispielsweise zu 70% gefüllt werden kann, was dadurch geschieht, dass aufgrund des unter Druck stehenden CO₂ der Spritzenkolben 44 im Aufnahmekörper 43, der sich zunächst spritzeneingangsseitig befindet, in Richtung B zurückbewegt wird. Ist dies erreicht, wird der Rasttaster 21 nochmals gedrückt, so dass das Schließventil 16 geschlossen wird. Wichtig ist, dass vor dem Aufbringen der Einheit 40 die Anschlussadapter 36 bzw. 37 mit dem jeweiligen Gas von anderen Gasen oder Fremdstoffen frei gespült werden. Dies geschieht dadurch, dass während einer bestimmten, vorher ermittelten Zeit, Gas durch die Leitungen geblasen wird, jedoch ohne dass die Einheit 40 (Sterilfilter 41 und Spritze 42) angeschlossen ist.

Danach wird die Einheit 40 vom Anschlussadapter 36 abgenommen und auf den Anschlussadapter 37 gebracht, worauf nach Umlegen des nicht dargestellten Wahlschalters von CO₂ auf O₂ und Betätigen des Rasttasters 21 dies dazu führt, dass der Aufnahmekörper 43 zusätzlich mit Sauerstoff befüllt wird, der unter Druck durch das Sterilfilter 41 gedrückt und unter weiterem Zurückschieben in Richtung B des Spritzenkolbens 44 den Aufnahmekörper 43 weiter, das heißt im Wesentlichen bis zum gewünschten Volumen vorzugsweise teilweise füllt. Auch hier werden vor dem Aufbringen der Einheit 40 die Anschlussadapter 36 bzw. 37 von Fremdgasen oder Fremdkörpern frei gespült, so wie vorab beschrieben. Damit ist das Befüllen der Spritze 42 bzw. deren Aufnahmekörper 43 mit hier zweierlei Gasen unterschiedlicher Volumina mittels der Vorrichtung 10 beendet. Dem Rasttaster 21 zugeordnet ist ein einstellbarer Schutzzeitschalter zur automatischen Beendigung des Füllvorganges falls der Rasttaster 21 beim zweiten Mal zu spät oder gar nicht betätigt wird.

Danach wird die Spritze 42 vom Sterilfilter 41 abgenommen und auf eine weitere mit einem Medikament teilweise befüllte Spritze mittels bereits bekannter Technik (über ein nicht näher beschriebenes Zwischenstück) gegengerichtet gesetzt, so dass sich durch Hin- und Herschieben der Kolben der jeweiligen Spritze das Medikament mit dem Gasgemisch mischt und beispielsweise aufschäumt, so dass diese Mischung aus Gasgemisch und Medikament bspw. mit der zweiten Spritze in den menschlichen oder tierischen Körper injiziert werden kann.

Jeweils vor dem Befüllen des Spritzen-Aufnahmekörpers 43 mit CO₂ bzw. O₂ wird die betreffende Abfülllinie innerhalb der Einheit 35 und des Behältnisses 11 mit dem betreffenden Gas zum Sterilisieren und Freiblasen von Fremdgasen, z.B. Luft gespült. Bei der ersten Abfülllinie (hier CO₂) erfolgt das Freiblasen der luftgefüllten Spritzentülle 45 bei aufgesteckter Spritze 42, deren Aufnahmekörper 43 danach zum Entleeren wieder abgenommen und danach zum Befüllen wieder aufgesteckt wird. Das Entleeren des Aufnahmekörpers 43 erfolgt durch Eindrücken des Kolbens 44 entgegen der Richtung B bis dieser an der Spitzentülle 45 anschlägt. Während dieses Vorgangs bleibt das Schließventil 16 geöffnet, damit kein Fremdgas in die Innenräume der Leitungen gelangt. Das Spülgas, hier CO₂, strömt solange in Strömungsrichtung A. Die zweite Abfülllinie (hier O₂) wird ohne aufgesteckte Spritze 42 mittels Durchleiten von O₂ leer und steril von Fremdgasen oder Fremdkörpern freigeblasen.

Die Vorrichtung 10 kann mit beliebigen, auch ortsfest installierten Druckgasquellen verbunden werden. Auch können die Entlastungsventile 29, 30 entfallen, da an den Druckgasquellen Handschließventile vorgesehen sind und die Entlastung der jeweiligen druckbehafteten Leitung über die Vorrichtung 10 selbst durchgeführt wird. In einem insbesondere transportablen Behältnis, das beispielsweise als mit einem aufklappbaren Deckel versehener Aktenkoffer ausgebildet ist, sind die Bauteile, wie Ventile, Schalter, Signallampen, elektrische Verbindungskabel u. dgl. der Vorrichtung 10 bzw. der Einheit 35 erschütterungsfrei gelagert.

Gemäß einer Variante kann jeweils ein Sterilfilter 41 Bestandteil des Ausganges 36, 38 bzw. 37, 38, d.h. ständig auf das freie Ende des Anschlussadapters 36 bzw. 37 aufgesteckt sein. Dabei wird das jeweilige spritzenseitige Ende mit einem vorher sterilisierten Stopfen verschlossen.

Es versteht sich, dass die Vorrichtung 10 auch für drei oder mehr Gase mit einer entsprechenden Vielzahl von Leitungssträngen und Schalterstellungen ausgebildet sein kann.

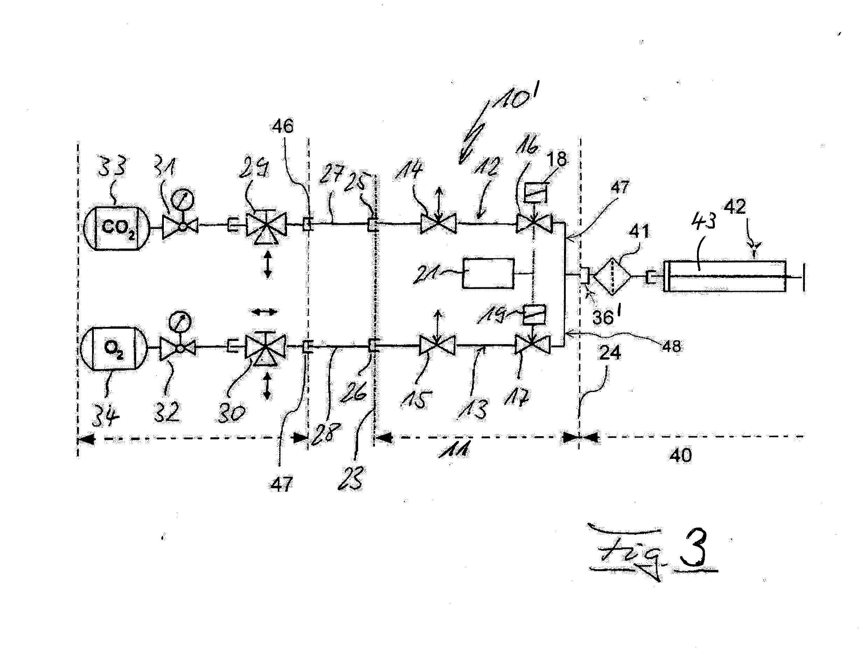

Beim Ausführungsbeispiel der Vorrichtung 10' nach Figur 3 wird nur ein Anschlussadapter 36' verwendet, der in die Seitenwand 23 des transportablen Behälters 11 feuchtigkeitsdicht eingebaut ist. Die beiden Leitungsstränge 47 bzw. 48 - von den Schließventilen 16 bzw. 17 kommend - führen über eine kurze Verbindung (T-Stück) direkt zum Anschlussadapter 36'. Dieses Ausführungsbeispiel, das ansonsten gleich der Vorrichtung 10 ist, hat den Vorteil, dass nur ein Anschlussadapter 36' und nur ein Sterilfilter 41 verwendet wird. Die Befüllung und Spülung mit Gas erfolgt wie früher zur Vorrichtung 10 beschrieben, jeweils zuerst über das Schließventil 16, hier im Beispiel mit CO₂ und anschließend über das Schließventil 17, hier im Beispiel mit O₂. Nachteilig gegenüber der Vorrichtung 10 nach Figur 1 ist, dass z.B. beim Füllen des zweiten Gases, hier O₂, noch Reste aus dem Leitungsstrang 47 ebenfalls über den Anschlussadapter 36' und das Sterilfilter 41 in die Spritze 42 gelangen und so zu einem etwas anderen, undefinierten Verhältnis der Gasmischung in der gefüllten Spritze 41 führen.

## Patentansprüche

1. Vorrichtung (10) zum Befüllen eines vorzugsweise Spritzenkörpers (43) mit einem Gasgemisch zum anschließenden Injizieren eines Gemisches aus dem Gasgemisch und einem Medikament in den menschlichen oder tierischen Körper in Anwendung für Diagnose und/oder Therapie, **gekennzeichnet durch** vorzugsweise zwei in getrennten Leitungssträngen (12, 13) angeordneten Ventilanordnungen aus jeweils einem Feinregulierventil (14, 15) und in Reihe geschaltetem Schließventil (16, 17) in einem vorzugsweise transportablen Behältnis (11), wobei das jeweils eine Ende der Leitungsstränge (12, 13) mit jeweils einem von vorzugsweise zwei Druckgasquellen und das andere Ende der Leitungsstränge (12, 13) getrennt oder gemeinsam mit einem mit dem oder den betreffenden Gasen zu füllenden vorzugsweise Spritzenkörper (43) an der Außenseite des Behältnisses (11) lösbar verbindbar ist.

2. Vorrichtung nach Anspruch 1, **dadurch gekennzeichnet, dass** das Feinregulierventil (14, 15) von Hand einstellbar ist.

3. Vorrichtung nach Anspruch 1 oder 2, **dadurch gekennzeichnet, dass** ein Schalter (21) zum Öffnen/Schließen eines aus beiden durch Aktivieren ausgewählten Schließventiles (16, 17) vorgesehen ist.

4. Vorrichtung nach Anspruch 3, **dadurch gekennzeichnet**, das der Schalter ein Rasttaster (21) ist, dem ein Schutzzeitschalter zugeordnet ist.

5. Vorrichtung nach mindestens einem der Ansprüche 1 bis 3, **dadurch gekennzeichnet, dass** jeweils eines der beiden Schließventile (16, 17) mittels eines Wahlschalters aktivierbar und das andere deaktivierbar ist.

6. Vorrichtung nach mindestens einem der vorhergehenden Ansprüche, **dadurch gekennzeichnet, dass** das Behältnis (11) ein tragbarer Aktenkoffer ist.

7. Vorrichtung nach Anspruch 6, **dadurch gekennzeichnet, dass** an parallelen Seitenwänden (23, 24) vorzugsweise senkrecht zur mit Scharnieren versehenen Rückwand des Aktenkoffers (11) kuppelbare bzw. steckbare Verbindungsanschlüsse (25, 26; 36, 37) vorgesehen sind.

8. Vorrichtung nach Anspruch 7, **dadurch gekennzeichnet, dass** das im Aktenkoffer (11) angeordnete Schließventil (16, 17) mit einem die Seitenwand (24) durchdringenden Anschlussadapter (36, 37) für ein Sterilfilter (41) und/oder Spritze (42) verbunden ist.

9. Vorrichtung nach Anspruch 7, **dadurch gekennzeichnet, dass** die im Aktenkoffer (11) angeordneten Schließventile (16, 17) gemeinsam mit einem die Seitenwand (24) durchdringenden Anschlussadapter (36') für ein Sterilfilter (41) und/oder Spritze (42) verbunden ist.

10. Vorrichtung nach mindestens einem der vorhergehenden Ansprüche, **dadurch gekennzeichnet, dass** an jedem der Anschlussadapter (36, 37) angeschlossen ein Sterilfilter (41) Bestandteil der Vorrichtung (10) ist.

11. Vorrichtung nach mindestens einem der vorhergehenden Ansprüche, **dadurch gekennzeichnet, dass** das Gasgemisch aus O₂ und CO₂ besteht, vorzugsweise im Verhältnis von 30% : 70%.
